# EUROPEAN PATENT APPLICATION

(11) **EP 1 479 398 A1**
(43) Date of publication of application: **24.11.2004**
(21) Application number: 03705018.4
(22) Date of filing: 23.01.2003
(51) Int. Cl.: A61K 48/00, A61K 38/17, A61K 45/00, A61P 35/00, A61P 43/00

(54) **AGENT CONTROLLING THE APOPTOSIS INDUCTION BY p73**

(30) Priority: 25.01.2002 JP 2002017486
(71) Applicant: HISAMITSU PHARMACEUTICAL CO., INC., Tosu-shi Saga 841-0017 (JP)
(72) Inventor: NAKAGAWARA, Akira, Chuo-ku, Chiba-shi, Chiba 260-0801 (JP)
(74) Representative: Grünecker, Kinkeldey, Stockmair & Schwanhäusser Anwaltssozietät
(86) International application number: PCT/JP2003/000605
(87) International publication number: WO 2003/061710

(57) **Abstract**

Elucidation of the interaction between p73 and ΔNp73 on the genetic and protein level, regulators for p73 or p53 apoptosis-inducing activity, a method of accelerating apoptosis of tumor cells by utilizing the regulators, p53 and p73 transactivation regulators comprising the p73 gene and ΔNp73 gene, nucleic acid pharmaceutical compositions comprising compositions for gene therapy which act as the regulators, and a method of screening for the regulators.

By forming a heterooligomer by a substance which binds with ΔNp73 promoter to p73 in an antagonistic manner in tumor cells (for example, nucleic acid including a base sequence which hybridizes to the base sequence listed as SEQ ID NO: 1) to control the promoter activity, it is possible to regulate the apoptosis-inducing activity of p73 and augment apoptosis of the tumor cells.

SEQUENCE LISTING

<110> Hisamitsu Pharmaceutical Co., Inc.

<120> Regulators for p73 apoptosis-inducing activity

<130> FP03-0002-00WO-HM

<140>

<141>

<150> JP 2002/17486

<151> 2002-01-25

<160> 17

<170> PatentIn Ver. 2.1

<210> 1

<211> 20

<212> DNA

<213> Homo sapiens

<400> 1

gggcaagctg aggcctgccc                                                    20

<210> 2

<211> 21

<212> DNA

<213> Artificial Sequence

<220>

<223> Primer

<400> 2

tggcttaccc atacgatgtt c                                                21

<210> 3

<211> 20

<212> DNA

<213> Artificial Sequence

<220>

<223> Primer

<400> 3

gtgctggact gctggaaagt                                                  20

<210> 4

<211> 20

<212> DNA

<213> Artificial Sequence

<220>

<223> Primer

<400> 4

tctggaacca gacagcacct                                                    20

<210> 5

<211> 20

<212> DNA

<213> Artificial Sequence

<220>

<223> Primer

<400> 5

gtgctggact gctggaaagt                                                    20

<210> 6

<211> 20

<212> DNA

<213> Artificial Sequence

<220>
<223> Primer

<400> 6
cgcctaccat gctgtacgtc                                                    20

<210> 7
<211> 20
<212> DNA
<213> Artificial Sequence

<220>
<223> Primer

<400> 7
gtgctggact gctggaaagt                                                    20

<210> 8

<211> 20

<212> DNA

<213> Artificial Sequence

<220>

<223> Primer

<400> 8

acctgacctg ccgtctagaa                                                    20

<210> 9

<211> 20

<212> DNA

<213> Artificial Sequence

<220>

<223> Primer

<400> 9

tccaccaccc tgttgctgta                                                    20

<210> 10

<211> 24

<212> DNA

<213> Artificial Sequence

<220>
<223> Primer

<400> 10
ggattcagcc agttgacaga acta                                              24

<210> 11
<211> 20
<212> DNA
<213> Artificial Sequence

<220>
<223> Primer

<400> 11
gtgctggact gctggaaagt                                                   20

<210> 12
<211> 20
<212> DNA
<213> Artificial Sequence

<220>

<223> Primer

<400> 12

ccagggagga tctgtagctg 20

<210> 13

<211> 20

<212> DNA

<213> Artificial Sequence

<220>

<223> Primer

<400> 13

tgaaccctac actgcagcaa 20

<210> 14

<211> 61

<212> DNA

<213> Artificial Sequence

<220>

<223> Probe

<400> 14

gtgcggtcca acacatcacc gggcaagctg aggcctgccc cggacttgga tgaatactca 60

t 61

<210> 15

<211> 300

<212> DNA

<213> Homo sapience

<400> 15

ggaacagagg cagcatctcg ccccctcccc gccccggact ggattgagcc ctgctttacg 60

gaaggagccc acgcagcctc tggatgaata agttgacaga gggtgagggc tggtgggttt 120

cttggtgcgg ctcatgagga actaagggag ctggggcccc aattatggag tccaacacat 180

ataaaggggt atgggaaaag ccgagccttc ccggcgctga caccgggcaa cccaggcagg 240

ccggcgtccc gctgaggcct gggccgcggg cgaaaatgcc ttttgttgtt aacaaacggc 300

<210> 16

<211> 20

<212> DNA

<213> Homo sapience

<220>

<221> misc_feature

<223> y=c, t, g

<400> 16
rrrcwwgyyy rrrcwwgyyy                                                    20

<210> 17
<211> 20
<212> DNA
<213> Homo sapience

<400> 17
gggcaagctg aggcctgccc                                                    20

## Description

### Technical Field

The present invention relates to tumor cell apoptosis regulators, and particularly to regulators for p73 or p53 apoptosis-inducing activity, and to a screening method for apoptosis regulators.

### Background Art

The p73 gene has been reported as a gene sharing high homology with the tumor suppressor gene p53 (Kaghad et al.). Unlike p53, however, p73 is expressed as multiple splicing variants. The transactivation regulating domain, DNA-binding domain and oligomerization domain present in p53 are conserved in both p73α and p73β (Kaghad et al., De Laurenzi et al., De Laurenzi and Catani et al.). Overexpression of the p73 gene has been observed to induce expression of, for example, the p21 gene which is induced by p53. Also, p73 has exhibited inhibition of tumor cell proliferation, acting as a transcription regulator similar to p53 (Kaghad et al., Jost et al.). Both p73 and p53 are believed to have sequence-specific transactivating functions, and although it has been thought that each recognizes and binds to the p53 responsive element in the promoter regions of the target genes, the precise mechanism has still not been elucidated.

The p73 gene is located at chromosome 1p36.2-3 which shows frequent loss of heterozygosity in a wide range of human tumors and its protein product induces cell cycle arrest or apoptosis, implying that it acts as a tumor suppressor similar to p53 (Kaghad et al., Schwab et al.). However, the fact that the p73 gene does not show the same high frequency of mutation as the p53 gene in cancers (Ikawa et al.), as well as other differences, suggest that the function of p73 is regulated by a different mechanism than the function of p53 (Lissy et al., Marin et al., Higashino et al., Steegenga et al., Haupt et al., Zeng et al.).

Incidentally, human neoplasms such as mammary carcinoma and ovarian carcinoma express higher levels of p73 than normal cells (Zaika et al., Chen et al.). Also, overexpression of cellular or viral oncogene products such as E2F-1, c-myc or E1A has been reported to induce expression of p73 (Lissy et al., Irwin et al., Stiewe et al., Zaika et al.). Thus, doubts remain as to whether the gene for p73 is a tumor suppressor gene or an oncogene.

Several mutants of the p73 protein exist, including ΔNp73 which lacks the N-terminal transactivation domain. Recently, Pozniak et al. discovered that apoptosis is inhibited by ΔNp73 blocking of the apoptosis-inducing activity of p53 in mouse sympathetic neurons (Pozniak et al.). These findings have suggested that ΔNp73, like p73, is an important regulating factor involved in cellular apoptosis.

As mentioned above, the function of p73 as a putative tumor suppressor is regulated by a complex mechanism in which ΔNp73 has been implicated, but which has not been fully understood to date. Elucidation of the apoptosis-inducing activity of p73 protein, including its mechanism, and the discovery of drugs to reinforce it, should lead to the development of new antitumor agents.

### Disclosure of the Invention

It is one of the objects of the present invention to elucidate the interaction between p73 and ΔNp73 on the genetic and protein level. It is another object of the invention to provide regulators for p73 or p53 apoptosis-inducing activity, a method of accelerating apoptosis of tumor cells using the regulators, and a method of screening for the regulators.

The present inventors have found that treatment of human neuroblastomas (hereinafter referred to simply as neuroblastomas) with cisplatin induces p53 and p73, while also inducing expression of ΔNp73. It was also found that the promoter region of ΔNp73 contains a sequence to which p73 binds specifically, and the sequence was determined. It was further found that overexpression of ΔNp73 inhibits p73-induced apoptosis, and the function of ΔNp73 as an autoregulatory factor for p73 was elucidated. It is believed that ΔNp73 forms a heterooligomer with p73, thereby inhibiting its transactivating function and apoptosis-inducing activity. Thus, p73-induced apoptosis is presumably controlled in tumor cells by the balance in intracellular levels of p73 and ΔNp73. Based on establishment of the molecular mechanism of interaction between p73 and ΔNp73 according to the present invention, it is possible to provide regulators for p73 or p53 apoptosis-inducing activity, a method of accelerating apoptosis of tumor cells using the regulators, regulators for p53 and p73 transactivation comprising the p73 gene and ΔNp73 gene, and a method of screening for the regulators.

In other words, the present invention relates to the following (1) to (7).
(1) A regulator for p73 apoptosis-inducing activity which utilizes heterooligomerization with ΔNp73.
(2) A regulator for p53 apoptosis-inducing activity which utilizes heterooligomerization with ΔNp73.
(3) A regulator for apoptosis-indicating activity which utilizes the base sequence set forth in SEQ ID NO: 1.
(4) A regulator for apoptosis-indicating activity consisting of a nucleic acid comprising a base sequence which hybridizes to the base sequence set forth in SEQ ID NO: 1.
(5) A p53 and p73 transactivation regulator comprising the p73 gene and the ΔNp73 gene.
(6) A method for screening an apoptosis regulator in a tumor cell which utilizes a nucleic acid having the base sequence set forth in SEQ ID NO: 1.
(7) A tumor cell apoptosis regulator obtainable by the method according to claim 6.

### Brief Description of the Drawings

Fig. 1 is a graph showing cisplatin-induced dose-dependent apoptosis of SH-SY5Y cells.
Fig. 2 is an immunoblot for cisplatin-treated SH-SY5Y cells.
Fig. 3 is an electropherogram showing the results of semiquantitative RT-PCR analysis of RNA from cisplatin-treated SH-SY5Y cells.
Fig. 4 is a Western blot of the product of SH-SY5Y cells infected with recombinant adenovirus expressing lacZ (Ad-lacZ), p53 (Ad-p53) or HA-p73α (Ad-p73α).
Fig. 5 is a Northern blot of the product of SH-SY5Y cells infected with recombinant adenovirus expressing lacZ (Ad-lacZ), p53 (Ad-p53) or HA-p73α (Ad-p73α).
Fig. 6 is an electropherogram showing the results of semiquantitative RT-PCR analysis of RNA from the products of SH-SY5Y, SK-N-AS and SK-N-BE cells infected with recombinant adenovirus expressing lacZ (Ad-lacZ), p53 (Ad-p53) and HA-p73α (Ad-p73α), respectively.
Fig. 7 is a graph showing the results of measuring ΔNp73 promoter expression by a luciferase reporter assay.
Fig. 8 shows the base sequence of the ΔNp73 promoter region.
Fig. 9 is a graph showing the results of cotransfection of SAOS-2 cells with pGL2-ΔNp73P(-100) and an expression plasmid (p53, HA-p63γ, HA-p73α, HA-p73β) and measurement of expression of the ΔNp73 promoter portion by a luciferase reporter assay.
Fig. 10 is a graph showing the results of cotransfection of SAOS-2 cells with pGL2-ΔNp73P(-76/-57) and an expression plasmid (p53, HA-p63γ, HA-p73α, HA-p73β) and measurement of expression of the ΔNp73 promoter portion by a luciferase reporter assay.
Fig. 11 is an electropherogram showing the results of testing binding reaction of competitive DNA which antagonistically binds with p73, with respect to ΔNp73 promoter, by electrophoretic mobility shift assay.
Fig. 12 is a electropherogram showing the results of the same type of electrophoretic mobility shift assay as in Fig. 11.
Fig. 13 is an immunoblot of the product of transfecting 293 cells with one or two expression plasmids (HA-p73α, HA-p73β, FLAG-ΔNp73α and/or FLAG-ΔNp73β).
Fig. 14 is an immunoblot of the product of transfecting COS7 cells with one or two expression plasmids (p53, FLAG-ΔNp73α and/or FLAG-ΔNp73β).
Fig. 15 is a graph showing the results of transfecting SAOS-2 cells with one or two expression plasmids (p53, p73α, p73β, ΔNp73α and/or ΔNp73β), and measuring expression of the MDM2 promoter by a luciferase reporter assay.
Fig. 16 is a graph showing the results of transfecting SAOS-2 cells with one or two expression plasmids (p53, p73α, p73β, ΔNp73α and/or ΔNp73β), and measuring expression of the Bax promoter by a luciferase reporter assay.
Fig. 17 is a graph showing the results of transfecting SAOS-2 cells with one or two expression plasmids (p53, p73α, p73β, ΔNp73α and/or ΔNp73β), and measuring expression of the ΔNp73 promoter by a luciferase reporter assay.
Fig. 18 is a graph showing changes in cell apoptosis as a result of coinfecting SK-N-BE cells with recombinant adenovirus expressing p73α (Ad-p73α) and increasing amounts of ΔNp73α (Ad-ΔNp73α).
Fig. 19 is a graph showing changes in cisplatin-induced cell apoptosis as a result of infecting SK-N-BE cells with recombinant adenovirus expressing ΔNp73α (Ad-ΔNp73α), and treating the product with cisplatin at variable concentration.

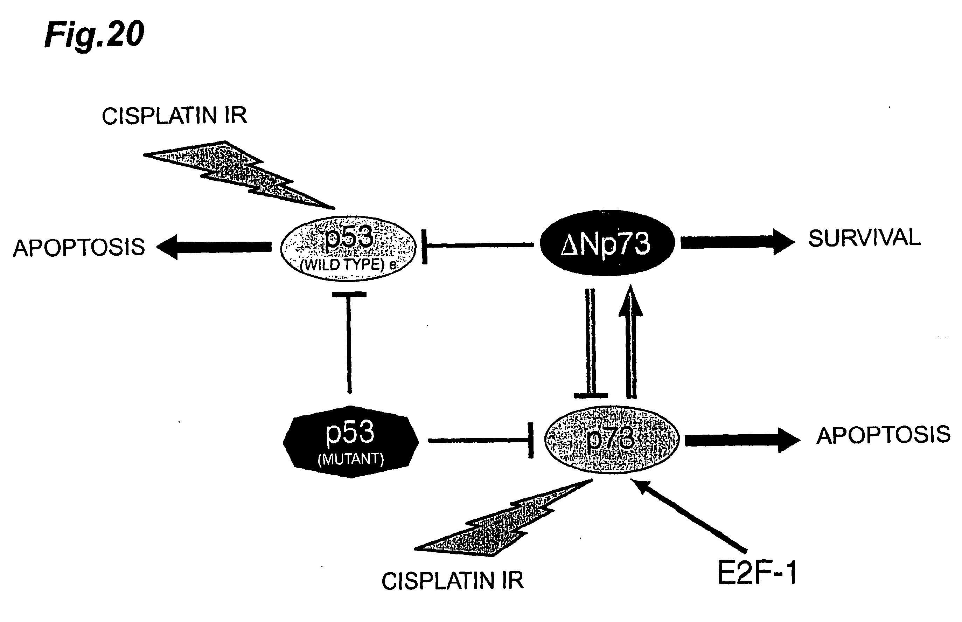

Fig. 20 is a schematic representation of the cellular level interactions and relationships of the tumor-related genes utilized for the invention.

### Best Mode for Carrying Out the Invention

Preferred modes of the present invention will now be explained in greater detail.

The term "nucleic acid" used throughout the present specification refers to DNA or RNA, for example, or to a polynucleotide comprising active DNA or RNA derived therefrom, and is preferably DNA or RNA. The nucleic acid most preferably has the DNA sequence disclosed in the present specification, or its complementary sequence.

The term "antagonistically-binding nucleic acid" used throughout the present specification refers to nucleic acid which binds competitively with the original target element of a binding sequence (nucleic acid or protein), and in its wide meaning includes antisense nucleic acid RNAi (RNA interference).

The term "hybridize" used throughout the present specification refers to forming a hybrid between nucleic acids under stringent conditions (see Maniatis et al., Molecular Cloning - A Laboratory Manual, Cold Spring Harbor Laboratory Press, New York, USA, 9.47-9.62 and 11.45-11.61, 1989) whereby the base sequence of interest is distinguished from unrelated base sequences, as understood by those skilled in the art. Examples of such stringent conditions include (1) using low ionic strength and high temperature for washing (for example, 50°C, 0.015 M sodium chloride, 0.0015 M sodium citrate, 0.1% SDS buffer), or (2) using a denaturing agent such as formamide during the hybridization (for example, 42°C, 50% formamide, 0.1% bovine serum albumin, 0.1% Ficol, 0.1% polyvinylpyrrolidone, 50. mM sodium phosphate buffer (pH 6.5), 750 mM sodium chloride, 75 mM sodium citrate). Another example is using 50% formamide, 5 x SSC, 50 mM sodium phosphate (pH 6.8), 0.1% sodium pyrophosphate, 5 x Denhardt's solution, sonicated salmon sperm DNA (50 µg/ml), 0.1% SDS and 10% dextran sulfate at 42°C, with washing in 0.2 x SSC and 0.1% SDS at 42°C. A person skilled in the art can easily determine and vary the stringent conditions as suited for obtaining a clearly detectable hybridization signal.

### Expression of p73 and ΔNp73 in tumor cells

The present inventors ascertained that treating tumor cells with the DNA inhibiting agent cisplatin causes apoptosis of the tumor cells in a dose-dependent manner. Production of proteins p53 and p73α was confirmed by Western blotting. The tumor cells in this case are preferably neuroblastoma cells, but may also be other central or peripheral nervous system tumor cells. Production of Δhp73α was also suggested by the aforementioned Western blotting, and this was confirmed by RT-PCR using a ΔNp73-specific primer. In other words, cisplatin induces production of p73α and ΔNp73α in tumor cells at both the mRNA level and the protein level.

### Relationship between p73 and ΔNp73

The present inventors also infected tumor cells with an adenovirus vector expressing genes for either p53 or p73α, and expression of ΔNp73α was found in the case of the p73α gene. This expression was confirmed by Western blotting, by Northern blotting using a ΔNp73-specific probe, or by RT-PCR using a ΔNp73-specific primer as above. In other words, p73α induces expression of ΔNp73α, whereas p53, unlike p73α, does not upregulate ΔNp73α.

### p73-specific sequence in ΔNp73

The present inventors further obtained full-length DNA for the ΔNp73 promoter and determined the base sequence of the p73-specific sequence. Continuous deletion analysis in the promoter region indicated that the position of the base sequence is -76 to -57 (SEQ ID NO: 1). A similar expression test using tumor cells showed, as expected, that transactivation of the ΔNp73 promoter occurs by p73α (or p73β) but not by p53. In addition, the results of a competitive assay test confirmed that the aforementioned base sequence region contributes to binding with p73 and is a p73-specific sequence.

### Interaction between p73 and ΔNp73

The present inventors also discovered that coinfection of tumor cells with adenovirus vectors expressing the p73 (α or β) gene and the ΔNp73 (α or β) gene results in interaction of both isoforms of ΔNp73 with p73α and p73β. Based on an assay using a reporter gene, both isoforms of ΔNp73 were shown to inhibit transactivation of p53 and p73 (α or β). It was also shown that ΔNp73 promoter activity is inhibited by p73 (α or β). Thus, p73 and ΔNp73 form a negative autoregulatory loop for regulation of transactivation.

### Regulation of p73 apoptosis-inducing activity by ΔNp73

The present inventors further discovered that coinfection of tumor cells with adenovirus vectors expressing the p73α gene and the ΔNp73 gene resulted in inhibition of apoptosis of the tumor cells in a dose-dependent manner. It was also found that cisplatin-induced apoptosis of tumor cells was inhibited by infection with the adenovirus vector expressing the ΔNp73 gene. Thus, the apoptosis-inducing activity of p73α is regulated by ΔNp73α on the cellular level.

### ΔNp73 gene having a regulating function on p73 apoptosis-inducing activity according to the invention (hereinafter referred to as "ΔNp73 gene of the invention") and protein encoded thereby (hereinafter referred to as "ΔNp73 protein of the invention")

As explained above, the invention provides the ΔNp73 gene and its encoded protein having a regulating function on p73 apoptosis-inducing activity. The regulating function is governed by the p73-specific binding base sequence disclosed by the present invention, in the promoter region upstream from the ΔNp73 gene. As a result of promotion or inhibition of this binding, the gene and protein regulate apoptosis of tumor cells through quantitative balance of p73 and its antagonist ΔNp73. The ΔNp73 gene can also be used for treatment or prevention of abnormal expression of p73 protein. The base sequence selected for such apoptosis regulation comprises the region upstream from the ΔNp73 gene, and preferably the ΔNp73 promoter region, with the p73-specific binding sequence preferably included in the region. The base sequence is preferably that listed as SEQ ID NO: 1, and most preferably, the specific binding sequence mentioned above is the DNA sequence listed as SEQ ID NO: 1. DNA having this base sequence can be easily obtained by chemical synthesis using an automatic DNA synthesizer.

The ΔNp73 gene of the invention may be linked to a suitable vector/promoter, transferred into host cells and used to produce its encoded protein which is then extracted and purified to obtain the protein of the invention. This procedure is well known to those skilled in the art. The ΔNp73 gene of the invention may be inserted into a suitable viral vector and the vector transferred into tumor cells for expression of the ΔNp73 protein of the invention under transcriptional control of a suitable promoter, for a regulating effect on tumor cell apoptosis. The expressed gene does not necessarily need to code for ΔNp73 protein. For example, a regulating effect on tumor cell apoptosis can be obtained by overexpressing an RNA transcript comprising a base sequence which can specifically bind to ΔNp73 mRNA (antisense or RNAi). The tumor cell apoptosis regulating effect also includes an effect of eliminating cisplatin resistance in cisplatin-resistant tumor cells. Elimination of resistance includes elimination of acquired non-MDR type drug resistance.

As suitable vectors there may be mentioned MoMLV vector, herpes virus vector, adenovirus vector, AAV vector, HIV vector, SIV vector and Sendai virus vector, with no limitation to these. Non-viral vectors may also be used, including liposomes, calcium phosphate and nucleic acid (transfer gene) complexes, cation-lipid complexes, Sendai virus liposomes, polymer carriers with polycations in the main chain, and the like. The gene may also be transferred into the cells by a method such as electroporation or using a gene gun.

The promoter used for expression of the gene inserted in the vector is not particularly restricted so long as it allows expression of the ΔNp73 gene of the invention in host cells such as tumor cells. For example, there may be mentioned promoters derived from viruses such as adenovirus, cytomegalovirus, HIV virus, Simian virus 40, Raus sarcoma virus, herpes simplex virus, mouse leukemia virus, sindbis virus, hepatitis A virus, hepatitis B virus, hepatitis C virus, papilloma virus, human T cell leukemia virus, influenza virus, Japanese encephalitis virus, JC virus, parvovirus B19 or polio virus, mammalian promoters such as albumin, SRα, heat shock protein, elongation factor and the like, chimeric promoters such as CAG promoter, and promoters subject to expression induction by tetracycline, steroids and the like. Promoters expressible in *E. coli,* such as *lac* promoter, may also be used.

When the ΔNp73 protein of the invention must be isolated, the isolation may be performed by a method publicly known to those skilled in the art. Examples include methods of homogenizing the host cells, methods of using a surfactant such as SDS or an enzyme to lyse the cell membranes, and methods of ultrasonic treatment and repeated freezing/thawing. When the amino acid length of the ΔNp73 protein of the invention is relatively short, an automatic peptide synthesizer may be used for chemical synthesis instead of the aforementioned gene recombination procedure. The ΔNp73 protein of the invention obtained by any of these methods may be purified by ordinary protocols. For example, common biochemical procedures such as ultracentrifugation or density gradient centrifugation, column separation using an ion-exchange column, affinity column or reverse phase column, gel separation using polyacrylamide, or the like may be used for purification.

As explained above, the invention also provides a method of controlling ΔNp73 promoter activity in the tumor cells to regulate p73 apoptosis-inducing activity for acceleration of tumor cell apoptosis. For this purpose, there is used nucleic acid (DNA) which can control ΔNp73 promoter activity by binding antagonistically to the ΔNp73 promoter region, and specifically to the p73-specific binding site (the base sequence listed as SEQ ID NO: 1).

For transfer of the DNA (hereinafter referred to as "DNA of the invention") into tumor cells, it is preferably administered to a patient as a nucleic acid pharmaceutical composition. A gene therapy method is used for this purpose. Specifically, the DNA of the invention to be used as a therapeutic gene is inserted into a gene transfer vector and delivered to target tumor cells. Appropriate recombinant vectors to be used for this purpose include the vectors capable of transferring genes into mammalian cells (especially human cells) among the vectors listed above. Likewise, promoters which may be used include those listed above which are promoters capable of expressing genes in mammalian cells (especially human cells). The recombinant vector incorporating the therapeutic gene may be dissolved in an appropriate solvent such as water, physiological saline, isotonized buffer or the like to prepare the composition for gene therapy.

DNA according to the invention is not necessarily essential to control ΔNp73 promoter activity. Ribozymes, RNAi and the like can control ΔNp73 gene expression and may also be used. The apoptosis regulator of the invention includes the aforementioned DNA, RNA and protein encoded by them.

### Screening method for tumor cell apoptosis regulators using nucleic acid with p73-specific binding base sequence

As explained above, the invention also provides a screening method for tumor cell apoptosis regulators which uses nucleic acid with the p73-specific binding base sequence.

Analytes for the screening method of the invention include not only proteins which bind to the ΔNp73 promoter or regulates its activity, or DNA coding therefor, but also compounds which regulate its activity. Such compounds may be synthetic or natural, low molecular or high molecular, or organic or inorganic.

The screening method of the invention may be carried out by any of various methods publicly known to those skilled in the art. For example, when the analyte is a non-protein, the analyte is contacted with cells (yeast, animal cells or the like) hosting a vector containing a reporter gene (for example, the luciferase gene) linked downstream from the ΔNp73 promoter, and it is determined whether or not reporter activity is regulated. Candidate compounds which control (especially inhibit) ΔNp73 promoter activity are then selected based on the reporter activity.

When the analyte is selected from a gene library, the gene library DNA is introduced into cells possessing the vector having a reporter gene (for example, the luciferase gene) linked downstream from the ΔNp73 promoter, and clones are selected which induce or, preferably, inhibit expression of the reporter gene. The clone DNA is sequenced and the protein encoded thereby is identified.

Also, ΔNp73 promoter DNA may be biotinylated and adsorbed onto streptavidin-bound beads or the like. These may then be incubated with the cell nuclear extract, and protein which binds specifically with the DNA selected by affinity purification. Alternatively, the ΔNp73 promoter DNA may be labeled and incubated with the cell nuclear extract, and incubation followed by electrophoretic mobility assay (also known as gel shift assay). If protein binding has occurred, the band of the DNA/protein complex after polyacrylamide gel electrophoresis will be shifted with respect to the band of the DNA alone, and this may be cut out from the gel and the protein extracted. Protein specifically binding to the DNA can be selected in this manner.

The protein is then expressed in gene library-introduced *E. coli*, and transferred to a filter. The ΔNp73 promoter DNA is used as a labeled probe, and blotted on the filter. Clones expressing protein which binds with the probe are selected. The DNA in the clones is sequenced and the protein encoded thereby identified.

Protein identified by this screening method as a tumor cell apoptosis regulator may be administered orally or parenterally to a patient (or warm-blooded animal) with a tumor, allowing the apoptosis regulator to act indirectly on the tumor cells. The apoptosis regulator is prepared as a pharmaceutical composition for this purpose. An effective dosage of the apoptosis regulator is combined with a pharmaceutically acceptable carrier or diluent to prepare a suitable dosage form. Suitable dosage forms for administration include tablets, pills, powders, solutions, suspensions, emulsions, capsules, suppositories, injections and the like.

The apoptosis regulator of the invention may be applied for a wide range of tumors in warm-blooded animals including humans, among which a typical example is neuroblastoma.

### (Examples)

The present invention will now be explained in greater detail by the following examples, with the understanding that the examples are in no way limitative on the invention.

### (Culturing and transfection or infection of cells)

Human neuroblastoma cells (SH-SY5Y, SK-N-AS and SK-N-BE) were cultured in RPMI-1640 medium containing 50 µg/ml kanamycin and 10% (v/v) heat-inactivated fetal bovine serum. Human osteosarcoma SAOS-2 cells, embryonic kidney 293 cells and COS7 cells were cultured in Dulbecco's Modified Eagle Medium (DMEM) containing 10% (v/v) heat-inactivated fetal bovine serum and antibiotics. The cultures were kept at 37°C in a humidified atmosphere containing 5% CO₂. The SAOS-2 cells were transfected with LipofectAMINE (GIBCO/BRL) according to the manufacturer's protocol. The 293 and COS7 cells were transfected with FuGENE 6 (Roche Molecular Biochemicals).

### (RNA Isolation and RT-PCR analysis)

Total RNA was prepared using Trizol reagent (GIBCO/BRL) or an RNeasy Mini Kit (Qiagen), according to the manufacturer's protocol. cDNA was prepared as template using 20 µl of cDNA synthesis reaction solution containing random primer and Moloney murine leukemia virus-reverse transcriptase (SuperScriptH, GIBCO/BRL), denaturation at 42°C for 1 hour followed by at 70°C for 15 minutes, and then rapid cooling. The cDNA was amplified in the 20 µl of PCR reaction solution containing 100 µl of each dNTP, 1 x PCR buffer, 10 µM of each primer and 0.2 unit of rTaq or LA-Taq DNA polymerase (TAKARA). The PCR primers used were 5'-TGGCTTACCCATACGATGTTC-3' (sense strand) (SEQ ID NO: 2) and 5'-GTGCTGGACTGCTGGAAAGT-3' (antisense strand) (SEQ ID NO: 3) for HA-p73; 5'-TCTGGAACCAGACAGCACCT-3' (sense strand) (SEQ ID NO: 4) and 5'-GTGCTGGACTGCTGGAAAGT-3' (antisense strand) (SEQ ID NO: 5) for p73; 5'-CGCCTACCATGCTGTACGTC-3' (sense strand) ) (SEQ ID NO: 6) and 5'-GTGCTGGACTGCTGGAAAGT-3' (antisense strand) (SEQ ID NO: 7) for ΔNp73; and 5'-ACCTGACCTGCCGTCTAGAA-3' (sense strand) (SEQ ID NO: 8) and 5'-TCCACCACCCTGTTGCTGTA-3' (antisense strand) (SEQ ID NO: 9) for GAPDH.

### (Plasmid and adenovirus mediated gene transfer)

Mammalian animal expression plasmids coding for p73α and p73β tagged with hemagglutinin (HA) were a gift from M. Kaghad. An expression plasmid for HA-p63γ was a gift from S. Ikawa. A 494 bp cDNA fragment coding for the NH₂-terminal region of ΔNp73 was amplified by RT-PCR using total RNA derived from SH-SYSY cells. The cells were infected with Ad-p73α. The primers used were 5'-GGATTCAGCCAGTTGACAGAACTA-3' (sense strand) (SEQ ID NO: 10) and 5'-GTGCTGGACTGCTGGAAAGT-3' (antisense strand) (SEQ ID NO: 11). The sense oligonucleotide primer was designed based on the same sequence found in both the human ΔNp73 genomic sequence and mouse ΔNp73 cDNA (Accession No.: Y19235). The amplified product was subcloned in pGEM-T Easy Vector (Promega) to obtain pGEM-ΔNp73. The identity of the construct was confirmed by DNA sequencing. pGEM-ΔNp73 was partially digested with NanrI and after blunting the ends, was fully digested with NanrI. The NH₂-terminal region restriction enzyme digestion fragment was subcloned at the enzymatically modified KpnI-NanrI site of pcDNA3-HA-p73α or pcDNA3-HA-p73β to obtain pcDNA3-ΔNp73α and pcDNA3-ΔNp73β, respectively. For construction of the adenovirus expression vectors, the HindIII-XbaI restriction enzyme digestion fragment from pcDNA3-p53, pcDNA3-HA-p73α, pcDNA3-HA-p73β or pcDNA3-ΔNp73α was filled in with Klenow fragment and inserted at the enzymatically modified NotI site of shuttle vector pHMCMV6 (Ozaki et al.). Each shuttle vector was digested with I-CeuI and PI-SceI and linked to the same restriction enzyme site of the adenovirus expression vector pAdHM4. Each of the recombinant adenovirus constructs was digested with PacI and used for transfection of 293 cells to produce recombinant adenovirus.

### (Immunoanalysis)

The cells were lysed in EBC buffer (50 mM Tris-HCl, pH 8.0/120 mM NaCl/0.5% Nonidet P-40) containing 1 mM phenylmethylsulfonyl fluoride. The lysate was clarified by centrifugation at 14,000 rpm at 4°C for 15 minutes. The protein concentration was determined by the Bradford procedure. For immunoblot analysis, the proteins were resolved by SDS polyamide gel electrophoresis and transferred to a nitrocellulose membrane. The membrane filter was blocked with 5% powdered milk in TBST (10 mM Tris-HCl, pH 8.0/150 mM NaCl/0.1% Tween 20) for 1 hour at room temperature. It was then incubated with monoclonal anti-HA (12CA5, Roche Molecular Biochemicals) antibody, monoclonal anti-p73α (Ab-1, Oncogene Research Products) antibody or monoclonal anti-p53 (DO-1, Oncogene Research Products) antibody for 1 hour at room temperature. The membrane filter was incubated with horseradish peroxidase-conjugated goat anti-mouse secondary antibody for 1 hour at room temperature. The bound secondary antibody was detected by enhanced chemiluminescence (ECL, Amersham Pharmacia Biotech) according to the manufacturer's protocol.

### (Immunoprecipitation)

293 cells or COS7 cells were transfected with 2 µg of the aforementioned expression plasmid. At 48 hours after transfection, the cells were lysed in 400 µl of EBC buffer, briefly sonicated and centrifuged at 14,000 rpm for 15 minutes at 4°C to remove the cell debris. After clarification, immunoprecipitation was carried out by incubating 400 µg of the whole cell lysates with a mixture of polyclonal anti-HA (Medical and Biological Laboratories) antibody or polyclonal anti-p53 (DO-1 and Pab1801, Oncogene Research Products) antibody. The immunocomplexes were precipitated with Protein A or Protein G Sepharose beads. The immunoprecipitates were washed 3 times with EBC buffer.

### (Cloning of ΔNp73 promoter region)

The ΔNp73 promoter region containing the ΔNp73 exon 3' was amplified by RT-PCR using PAC clone (dJ363H11) and the primers 5'-CCAGGGAGGATCTGTAGCTG-3' (sense strand) (SEQ ID NO: 12) and 5'-TGAACCCTACACTGCAGCAA-3' (antisense strand) (SEQ ID NO: 13). The amplified PCR product (2.9 Kb) was directly cloned in pGEM-T Easy Vector (Promega) to obtain pGEM-ΔNp73. The sequence of the construct was confirmed by DNA sequencing. In order to generate a series of 5'-deletion constructs, the 2.9 Kb fragment was subcloned at the enzymatically modified XhoI site of a pGL2 basic luciferase reporter (Promega), to obtain pGL2-ΔNp73PF. pGL2-ΔNp73PF was digested with SmaI, NcoI or StuI, blunt ended and self-ligated to produce pGL2-ΔNp73P(-1082), pGL2-ΔNp73P(-911) or pGL2-ΔNp73P(-63). Also, pGL2-ΔNp73PF was partially digested with ApaI and the restriction enzyme digestion fragments were collected, blunt ended and self-ligated to produce pGL2-ΔNp73PF(-1245), pGL2-ΔNp73PF(-786), pGL2-ΔNp73P(-619) and pGL2-ΔNp73P(-203). In order to generate ΔNp73P(-184), ΔNp73P(-100), ΔNp73P(-80), ΔNp73P(-76), ΔNp73P(-71), ΔNp73P(-66) or ΔNp73P(+1), the corresponding regions were amplified by PCR using pGEM-ΔNp73P as template. The obtained PCR products were subcloned at the SmaI site of the pGL2 basic luciferase reporter and the nucleotide sequences of the constructs were confirmed by sequencing.

### (Electrophoretic mobility shift assay)

A TNT Quick Coupled Transcription/Translation System (Promega) was used according to the manufacturer's instructions for *in vitro* production of p73α, p73β or p53. The electrophoretic mobility shift assays were performed as described previously (Ozaki et al.) Briefly, the doublestranded oligonucleotide was ³²P-labeled using T4 polynucleotide kinase. DNA-protein binding was carried out at room temperature in a reaction mixture comprising 4 µl of reticulocyte lysate, 12.5 mM Tris-HCl (pH 7.5), 50 mM KCl, 3.125 mM MgCl₂, 0.25 mM EDTA, 0.5 mM DTT, 5% glycerol and 100 µg/ml poly(dI-dC). The reaction mixture was resolved at room temperature on 5% polyacrylamide gel, in 1 x Tris-Borate-EDTA buffer.

### (Luciferase reporter assay)

For the luciferase reporter assays, SAOS-2 cells (p53 homozygous deleted) were seeded in triplicate into 12-well plates (5 x 10⁴ cells/well) 24 hours prior to transfection. The cells were cotransfected with 200 ng of reporter plasmid, 20 ng of pRL-TK encoding *Renilla* luciferase cDNA and 200 ng of expression plasmid (p53, p73α or p73β), in the presence or in the absence of ΔNp73α or ΔNp73β. The total amount of transfected DNA was kept constant with pcDNA3 (Invitrogen). At 48 hours after transfection, the luciferase activity was assayed using a Dual-Luciferase Reporter Assay System (Promega). The transfection efficiency was standardized against *Renilla* luciferase activity.

### (MTT survival assay)

SH-SY5Y and SK-N-BE cells were seeded into 96-well plates (5 x 10³, 2 x 10⁴ cells/well, respectively) 24 hours before virus infection. The cells were infected using Ad-lacZ or Ad-p73α at the indicated MOI, for prescribed time periods. The cell survival rates were determined by MTT assay. The substrate used in this case was (2-(2-methoxy-4-nitrophenyl)-3-(4-nitrophenyl)-5-(2,4-disulfophenyl)-2H-tetrazolium monosodium salt.

### (Example 1) p73 and ΔNp73 expression induction by cisplatin in neuroblastoma cells

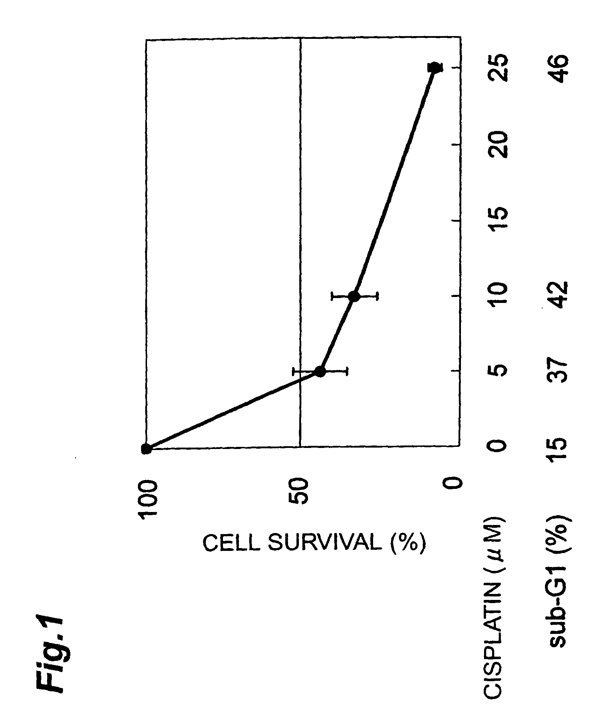

Neuroblastoma SH-SY5Y cells were cultured for 24 hours in the presence of cisplatin (0, 5, 10 and 25 µM concentrations). Determination of the surviving cell count by MTT assay revealed a reduction in surviving cells which was dependent on cisplatin dose. The data are shown in Fig. 1, as averages (±SD) for three experiments. The number of cells with sub-G1 DNA content was also counted, and the data are shown in Fig. 1.

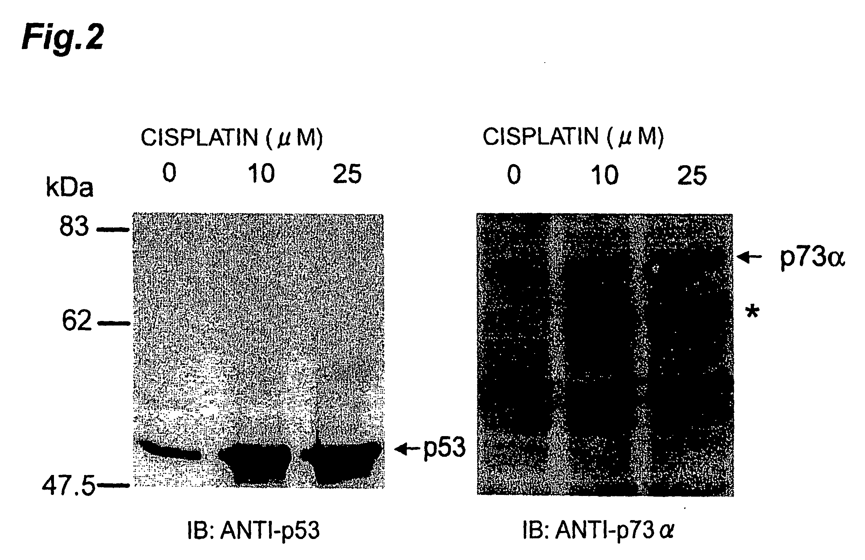

Next, SH-SY5Y cells were cultured for 36 hours in the presence of cisplatin (0, 10 and 25 µM concentrations). The whole cell protein (40 µg) was extracted from each culture. The expression levels of p73α and p53 were each determined by immunoblot analysis (described above) using p73α-specific antibody (Ab-1) or p53-specific antibody (DO-1). The results are shown in Fig. 2. The size marker is shown at left in the drawing. The "*" symbol indicates the band with faster mobility than p73α, which was recognized by the p73α-specific antibody and matches the estimated molecular weight of ΔNp73α (62 kDa; Polzniak et al.).

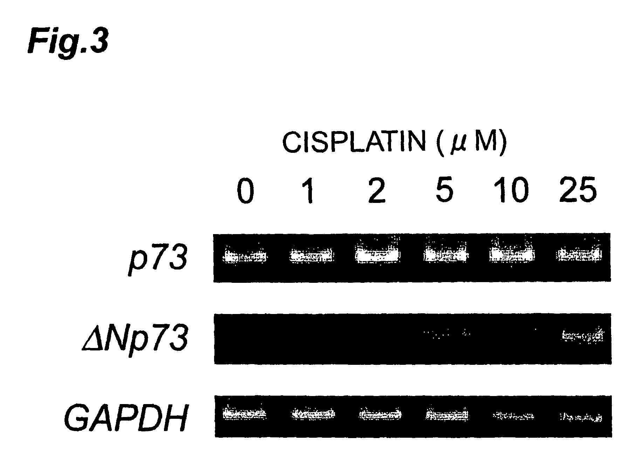

SH-SYSY cells were also cultured for 24 hours in the presence of cisplatin (0, 1, 2, 5, 10 and 25 µM concentrations). After culturing, the total RNA was prepared and used for semiquantitative RT-PCR (described above). The PCR reaction solution was electrophoresed on 2.5% agarose gel. The results are shown in Fig. 3, which also shows GAPDH expression as the control. The results in Fig. 3 demonstrate that expression of ΔNp73α increases in a cisplatin dose-dependent manner.

### (Example 2) Induction of ΔNp73 expression by p73α

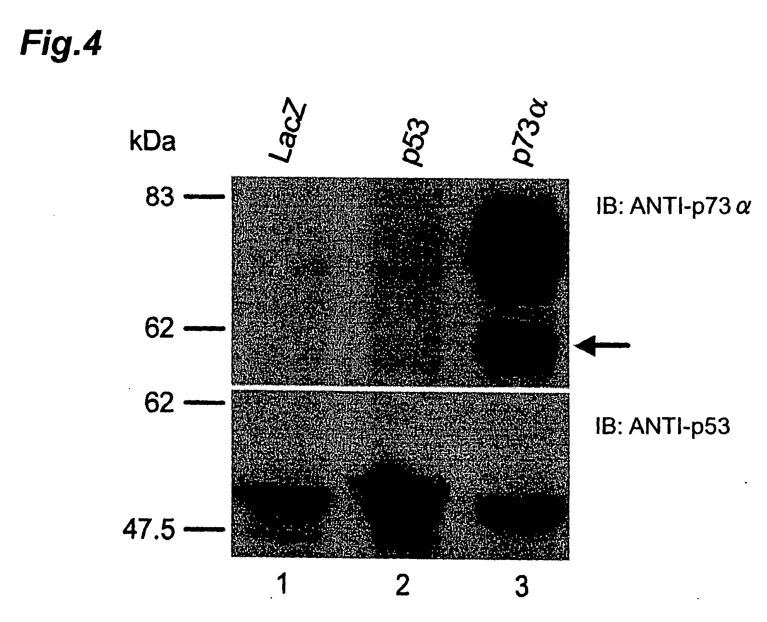

SH-SY5Y cells were infected with a recombinant adenovirus expressing lacZ (Ad-lacZ), p53 (Ad-p53) or HA-p73α (Ad-p73α) (MOI: 10). Whole cell lysate (40 µg) was prepared 36 hours after infection, and anti-p73α antibody or anti-p53 antibody was used for Western blotting. The results are shown in Fig. 4. Here, lane 1 corresponds to Ad-lacZ, lane 2 corresponds to Ad-p53 and lane 3 corresponds to Ad-p73α. As this image clearly shows, expression of HA-p73α induces expression of ΔNp73, while expression of p53 does not.

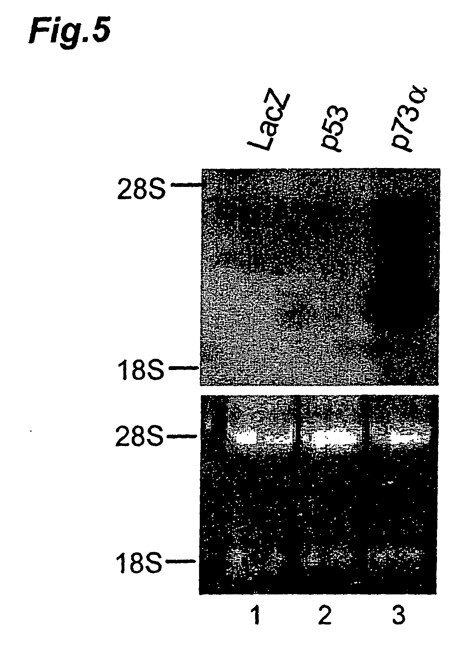

SH-SY5Y cells were also similarly infected with a recombinant adenovirus expressing lacZ (Ad-lacZ), p53 (Ad-p53) or HA-p73α (Ad-p73α) (MOI: 10). Total RNA (20 µg) was prepared 36 hours after infection, but in this case, a ΔNp73-specific probe was used for Northern blotting. The results of ethidium bromide staining are shown in Fig. 5. Here, lane 1 corresponds to Ad-lacZ, lane 2 corresponds to Ad-p53 and lane 3 corresponds to Ad-p73α. In this case as well, induction of ΔNp73 expression was confirmed in lane 3.

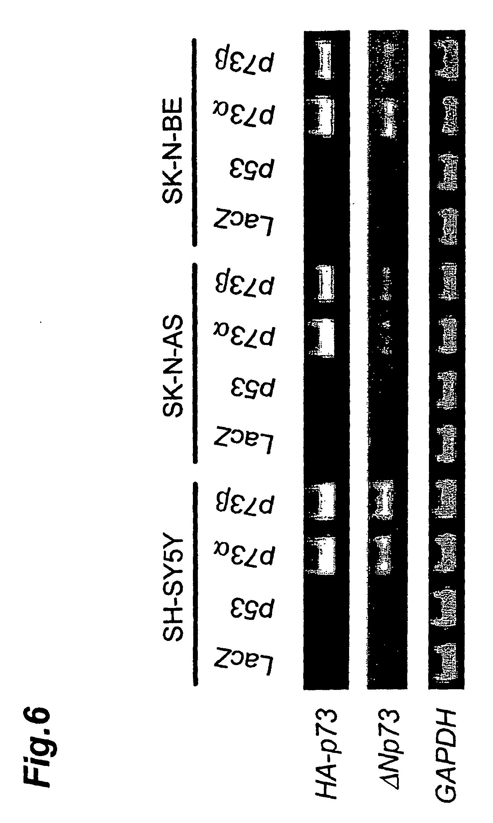

Total RNA was prepared from the above-mentioned adenovirus-infected (MOI: 10) SH-SYSY, SK-N-AS and SK-N-BE cells, and RT-PCR (described above) was performed. The results are shown in Fig. 6, which also shows GAPDH expression as the control. The results were the same as in the previous experiment, and clearly demonstrated induction of ΔNp73 expression by p73α and p73β.

These experimental results indicate that p73α upregulates ΔNp73α.

### (Example 3) Identification of p73-specific binding sequence in ΔNp73 promoter region

### (Luciferase reporter assay)

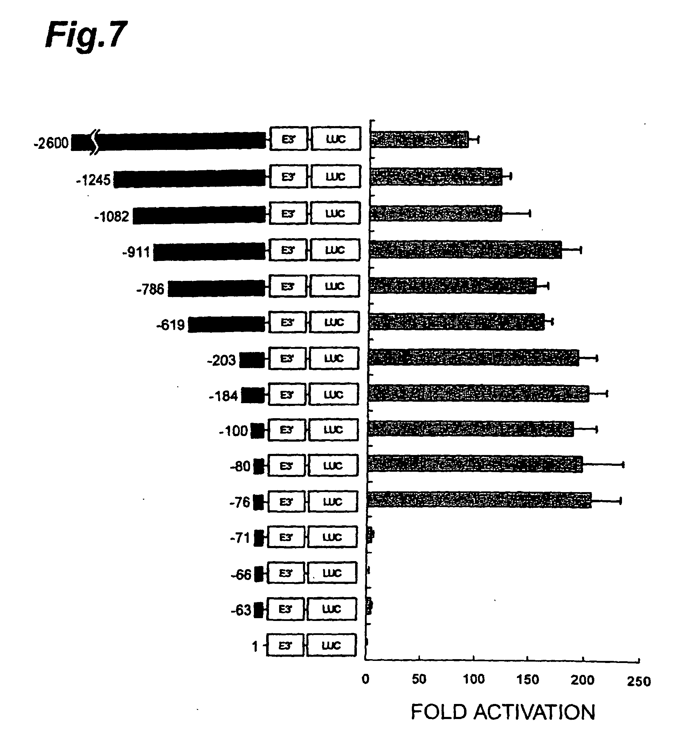

The luciferase reporter constructs pGL2-ΔNp73P(-2600), pGL2-ΔNp73P(-1245), pGL2-ΔNp73P(-1082), pGL2-ΔNp73P(-911), pGL2-ΔNp73P(-786), pGL2-ΔNp73P(-619), pGL2-ΔNp73P(-203), pGL2-ΔNp73P(-184), pGL2-ΔNp73P(-100), pGL2-ΔNp73P(-80), pGL2-ΔNp73P(-76), pGL2-ΔNp73P(-71), pGL2-ΔNp73P(-66), pGL2-ΔNp73P(-63) and pGL2-ΔNp73P(+1) were created in the manner described above. Each of the constructs was used for a luciferase reporter assay (described above), for coexpression with p73α. The results of the assay are shown in Fig. 7. The data represent the averages (±SD) for at least three experiments. Construct pGL2-ΔNp73P(-2600) containing the 2.6 kb sequence upstream from the 3' exon exhibited an increase in luciferase expression of about 90-fold in comparison to the vector lacking the promoter. These results demonstrated that deletion up to position -71 notably quenches luciferase activity.

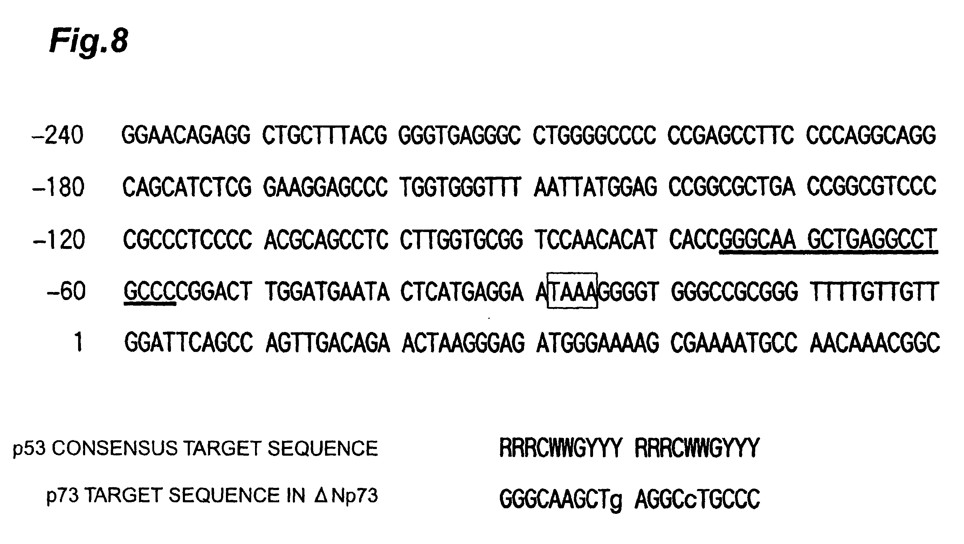

Fig. 8 shows the base sequence of the ΔNp73 promoter region. The putative p73-binding site is underlined, and the putative TATA element is boxed. The drawing also shows a sequence comparison between the consensus p53 target sequence and the putative p73-binding sequence. Here, R stands for purine, Y stands for pyrimidine, and W stands for adenine or thymidine. The two mismatches are represented by lowercase letters (c, g).

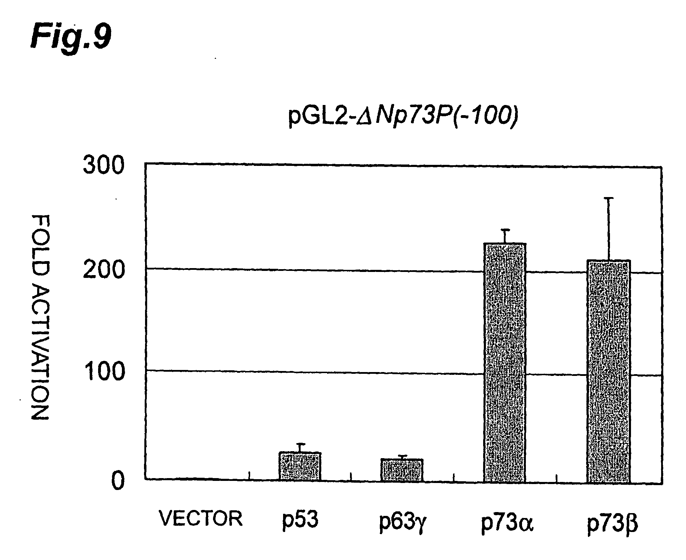

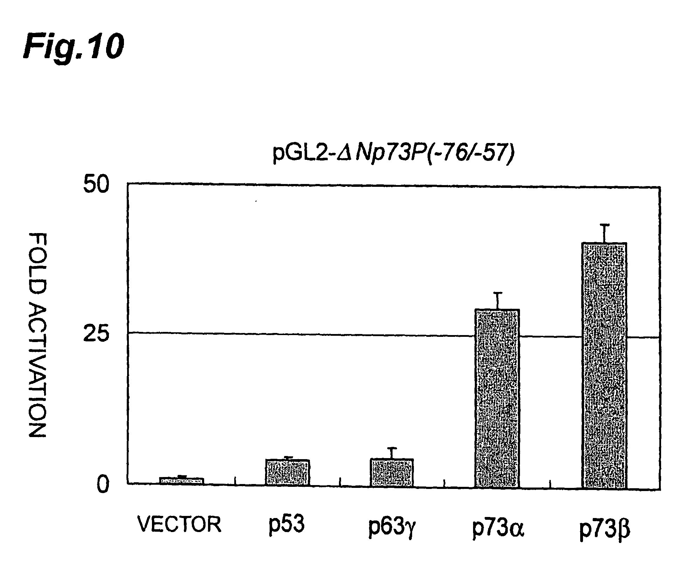

Since it was believed that the putative p73-binding site is essential for activation of the ΔNp73 promoter, the 20 bp sequence (5'-GGGCAAGCTGAGGCCTGCCC-3') (SEQ ID NO: 1) was subcloned in the region upstream from the luciferase plasmid pGL2 promoter to obtain pGL2-ΔNp73P(-76/-57). SAOS-2 cells were cotransfected (as described above) with 200 ng of reporter plasmid pGL2-ΔNp73P(-100) or pGL2-ΔNp73P(-76/-57), 20 ng of pRL-TK encoding *Renilla* luciferase cDNA and 100 ng of expression plasmid (p53, p63γ, HA-p73α or HA-p73β). The data for the luciferase activity assay are shown in Fig. 9 (pGL2-ΔNp73P(-100)) and Fig. 10 (pGL2-ΔNp73P(-76/-57)), as averages (±SD). The results shown in these graphs indicate, as expected, that p73α and p73β notably augmented luciferase expression by pGL2-ΔNp73P(-76/-57), while p53 and p63γ both had low degrees of augmentation. This demonstrated that the p73-binding element comprising the 20 bp sequence participates in p73-specific activation of the ΔNp73 promoter.

### (Electrophoretic mobility assay)

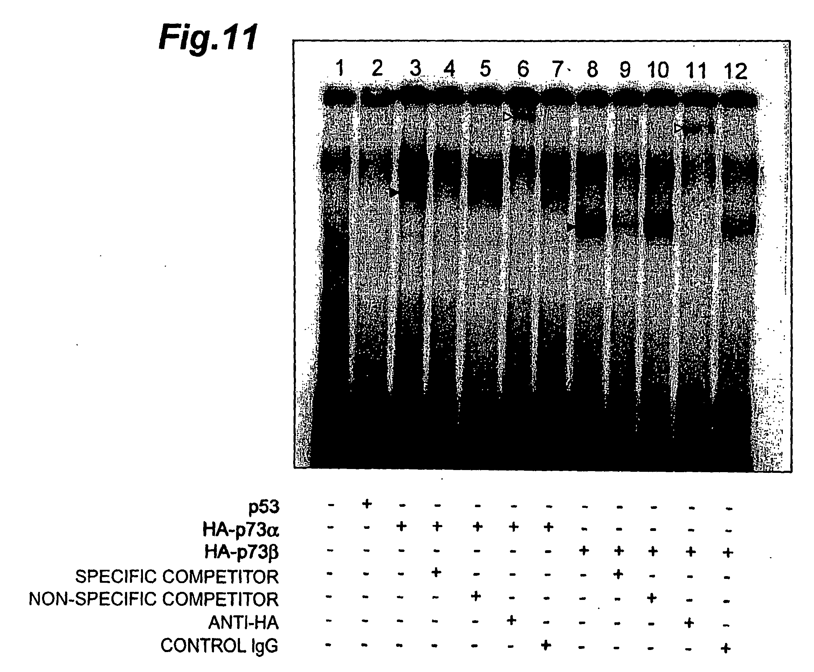

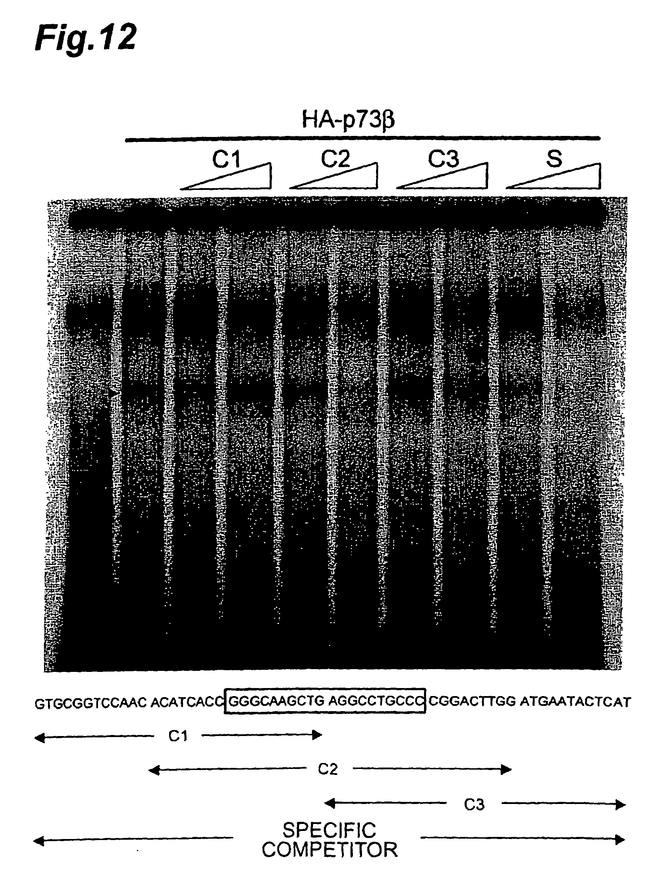

Sequence-specific competitive DNA and non-sequence-specific competitive DNA were used for blocking of the p73-specific binding sequence for further confirmation of the role of the p73-binding element represented by the sequence. A 61 bp oligonucleotide fragment comprising the p73-specific binding sequence (5'-GTGCGGTCCAACACATCACCGGGCAAGCTGAGGCCTGCCCCGGACTTGGATGAATACTCA T-3') (SEQ ID NO: 13) was labeled with [Υ³²P]ATP, and incubated with in vitro translated p53 (lane 2 in Fig. 11), HA-p73α (lanes 3-7), HA-p73β (lanes 8-12) and non-programmed reticulocyte lysate (lane 1). The results are shown in Fig. 11. Lanes 4 and 9 represent the results of the binding reaction with a 10-fold molar excess of the sequence-specific competitive DNA. Lanes 5 and 10 represent the results of the binding reaction with a 10-fold molar excess of the non-sequence-specific competitive DNA. The non-sequence-specific competitive DNAs comprised only the 5'- or 3'-end portion of the p73-specific binding sequence, and they are represented by C1 and C3 in Fig. 12 explained below. Anti-HA antibody was added to the reaction mixture (lanes 6 and 11) for supershifting of the protein/DNA complex. Preimmunoserum was used as a negative control (lanes 7 and 12). The protein/DNA complex and the supershifted complex are shown as wedges (solid or white, respectively). Both p73α and p73β bound specifically to the oligonucleotide fragment.

Reaction solutions containing in vitro translated HA-p73β (lanes 2-10) or non-programmed reticulocyte lysate (lane 1) were subjected to electrophoretic mobility assay in the same manner, yielding the results shown in Fig. 12. The sequence of the non-labeled competitive oligonucleotide used is shown at the bottom, with the p73-specific binding sequence boxed. The competitive oligonucleotides (DNA) were used at 1-fold or 10-fold excess, and are indicated by ramps at the top. The position of the protein/DNA complex is indicated by a solid wedge. As this image clearly shows, the C2 competitive DNA which contained the entire p73-specific binding sequence competed efficiently for p73β-DNA complex formation. Competition was lost, however, with the C1 competitive DNA and C3 competitive DNA. Similar results were obtained for p73β as well (not shown).

These data demonstrate that binding of p73 to this base sequence within the ΔNp73 promoter activates transcription of ΔNp73.

### (Example 4) Interaction between p73 and ΔNp73

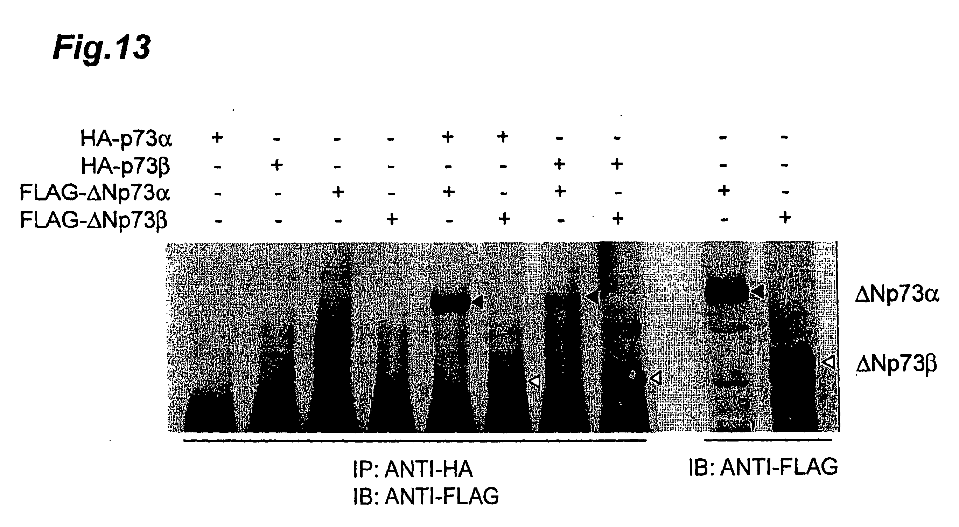

293 cells were transiently transfected with one or two expression plasmids (HA-p73α, HA-p73β, FLAG-ΔNp73α and/or FLAG-ΔNp73β). The whole cell lysates were immunoprecipitated with HA antibody (see above). The precipitated protein was subjected to immunoblot analysis with anti-FLAG M2 antibody, yielding the results shown in Fig. 13. Here, ΔNp73α and ΔNp73β are indicated by wedges (solid and white, respectively). It was demonstrated that both isoforms of ΔNp73 interact with p73α and p73β.

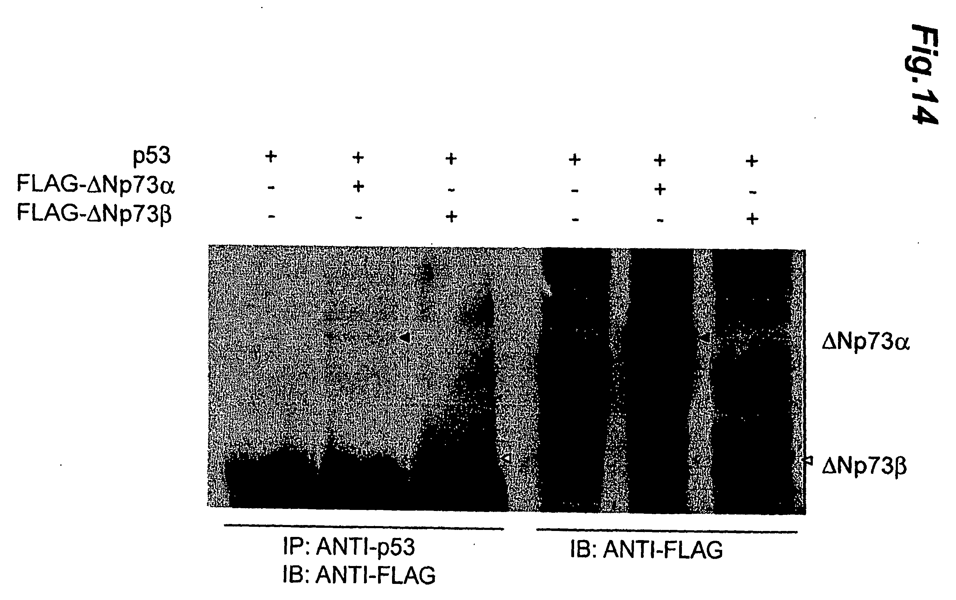

COS7 cells were transiently transfected with one or two expression plasmids (p53, FLAG-Np73 and/or FLAG-Np73β). Whole cell lysate was prepared 48 hours after transfection and immunoprecipitated with anti-p53 (DO-1/Pab1801), and then subjected to immunoblot analysis with anti-FLAG M2 antibody. The results are shown in Fig. 14. Here, ΔNp73α and ΔNp73β are indicated by wedges (solid and white, respectively). It was demonstrated that p53 interacts with p73α and p73β.

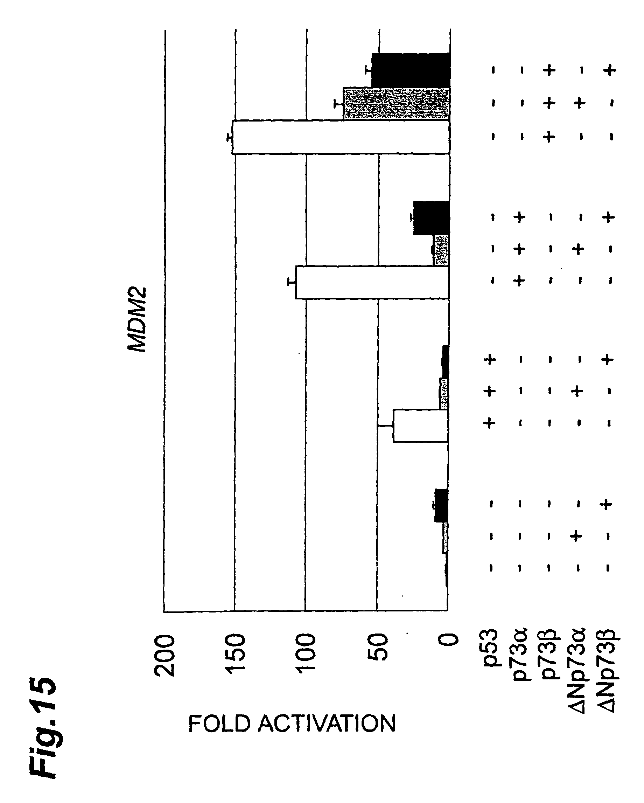

Next, interaction between p73 and ΔNp73 was confirmed by a luciferase reporter assay (see above). SAOS-2 cells were cotransfected with one or two expression plasmids (p53, p73α, p73β, ΔNp73α and/or ΔNp73β) and a reporter plasmid containing the MDM2 promoter. Luciferase assay was performed 48 hours after transfection, yielding the results shown in Fig. 15. The data represent averages (±SD).

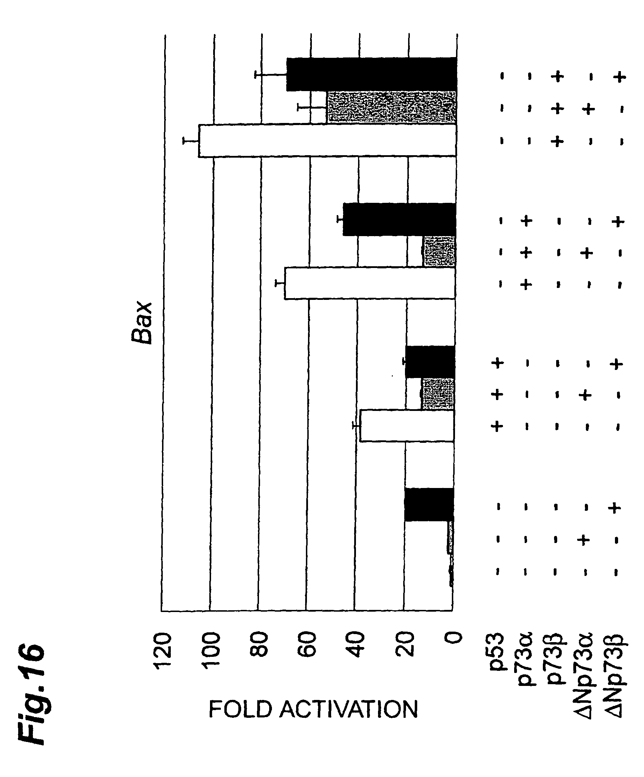

SAOS-2 cells were similarly cotransfected with expression plasmids (p53, p73α, p73β, ΔNp73α and ΔNp73β and a reporter plasmid containing the Bax promoter. Luciferase assay was performed 48 hours after transfection, yielding the results shown in Fig. 16. The data represent averages (±SD). The data in Figs. 15 and 16 demonstrate that transactivation of the MDM2 promoter and the Bax promoter induced by p73α or p73β is hindered by ΔNp73α or ΔNp73β.

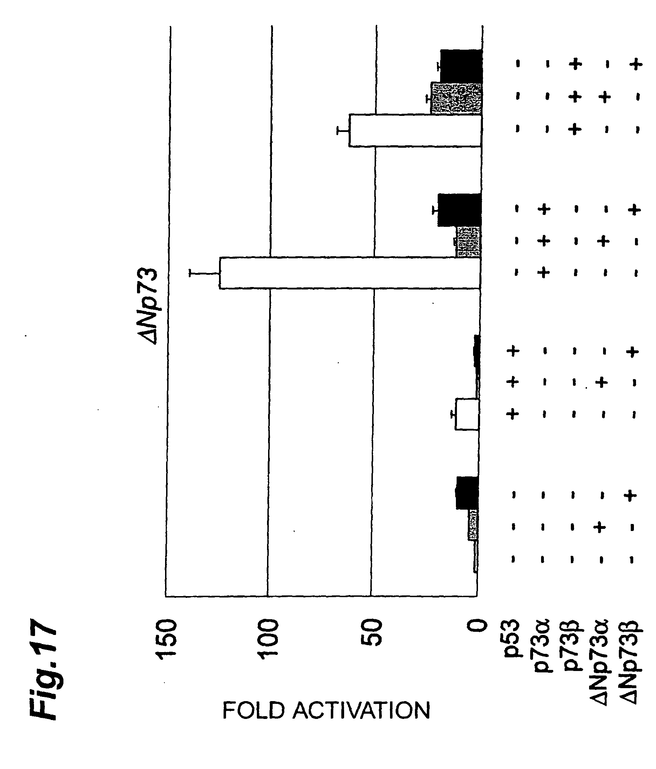

SAOS-2 cells were also cotransfected with one or two expression plasmids (p53, p73α, p73β, ΔNp73α and/or ΔNp73β) and a reporter plasmid containing the ΔNp73 promoter. Luciferase assay was performed 48 hours after transfection, yielding the results shown in Fig. 17. The data represent averages (±SD). It was demonstrated that ΔNp73 promoter activity is notably reduced by coexpression of ΔNp73α or ΔNp73β with p73α or p73β.

These results concur with the fact that increasing the p73α protein level reduces the level of ΔNp73 expression in a dose-dependent manner (data not shown).

### (Example 5) Inhibition of p73- or cisplatin-induced apoptosis by ΔNp73

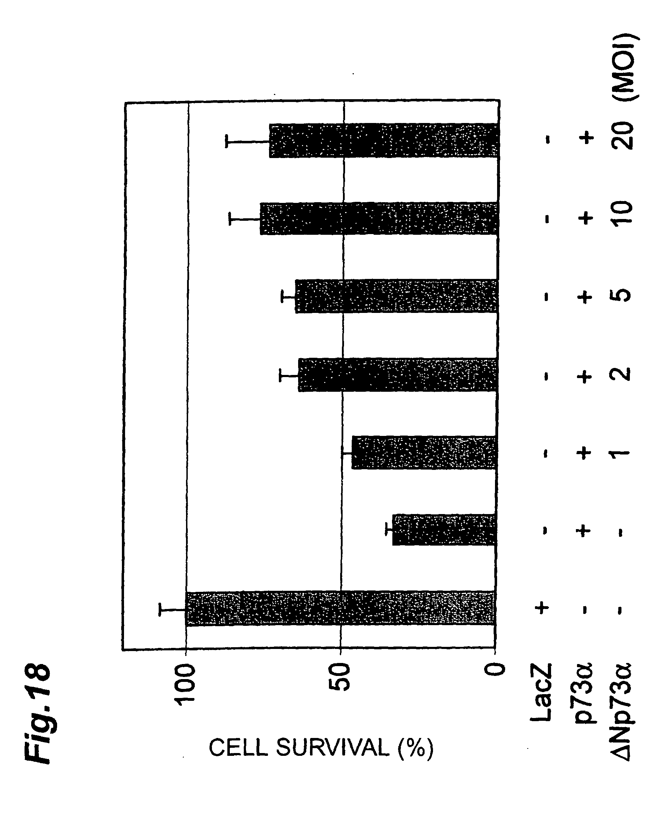

SK-N-BE cells (2 x 10⁴/well) were coinfected with Ad-p73α (MOI: 10) and Ad-ΔNp73α (0, 1, 2, 5, 10, 20 MOI). The number of surviving cells was measured by MTT survival assay 48 hours after infection. The results are shown in Fig. 18. The graph shows the relative surviving cell percentage compared to infection with a lacZ control (average of 6 experiments (±SD)). It was demonstrated that increasing the level of ΔNp73α reduces p73α-induced apoptosis.

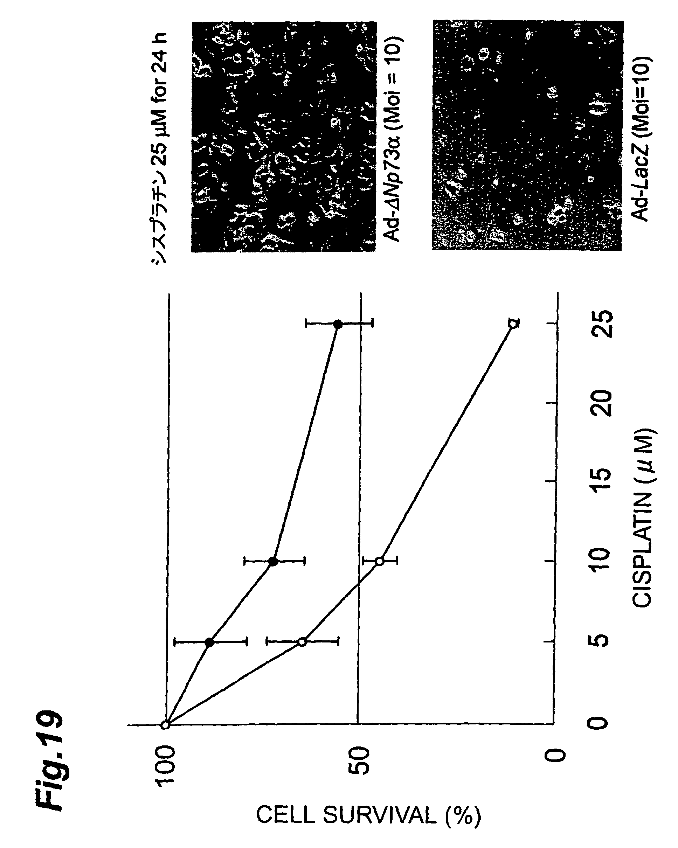

SH-SY5Y cells (5 x 10³/well) were infected with Ad-lacZ or Ad-ΔNp73α (MOI: 10). At 6 hours after infection, the cells were treated with cisplatin (0, 5, 10 and 25 µM concentration) for 24 hours. The number of surviving cells was measured by MTT survival assay, yielding the results shown in Fig. 19. The graph shows the averages of 6 experiments (±SD). Photographs of the cells after infection with Ad-lacZ or Ad-ΔNp73α (both MOI: 10) and treatment with 25 µM cisplatin for 24 hours are shown at the right of the drawing. It was demonstrated that cisplatin-induced apoptosis of SH-SY5Y cells is inhibited by ΔNp73α infection.

### (Cited Publications)

1. Kaghad, M., Bonnet, H., Yang, A., Creancier, L., Biscan, J. C., Valent, A., Minty, A., Chalon, P., Lelias, J. M., Dumont, X., Ferrara, P., MeKeon, F. & Caput, D. (1997) Cell 90, 809-8 19.
2. De Laurenzi, V., Costanzo, A., Barcaroli, D., Terrinoni, A., Falco, M., Annicehiarico-Petruzzelli, M., Leyrero, M. & Melino, G. (1998) J. Exp. Med. 188, 1763-1768.
3. De Laurenzi, V., Catani, M., Costanzo, A., Terrinoni, A., Corazzari, M., Levrero, M., Knight, R. & Melino, G. (1999) Cell Death Differ. 6,389-390.
4. Jost, C., Mann, M. & Kaelin, W. G., Jr. (1997) Nature 389, 19 1-194. 5. Schwab, M., Prarni, C. & Amler, L. C. (1996) Genes Chromosomes Cancer 16, 211-229.
6. Ikawa, S., Nakagawara, A. & Ikawa, Y (1999) Cell Death Dffer. 6, 1154-1161.
7. Lissy, N. A., Davis, P. K., Irwin, M., Kaelin, W. G. J- & Dowdy, S. F. (2000) Nature 407, 642-645. 21.
8. Marin, M. C., Jost, C. A., Irwin, M. S., DeCaprio, J. A., Caput, D. & Kaelin, W. G. Jr. (1998) Mci. Cell. Bid. 18, 6316-6324.
9. Higashino, F., Pipas, J. M. & Shenk, T. (1998) Proc. Nati. Acad. Sci. USA 95, 15683-15687.
10. Steegenga, W. T., Shyarts, A., Riteco, N., Bos, J. L. & Jochernsen, A. G. (1999) Mel. Cell. Bid. 19, 3 885-3894.
11. Haupt, Y., Maya, R., Kazaz, A. & Oren, M. (1997) Nature 387,296-299.
12. Zeng, X., Chen, L., Jost, C. A., Maya, R., Keller, D., Wang, X., Kaelin, W. G. Jr., Oren, M., Chen, J. & Lu, H. (1999) Mel. Cell. Bid. 19, 3257-3266.
13. Zaika, A. I., Kovalev, S., Marchenko, N. & Moll, U. M. (1999) Cancer Res. 59, 3257-3263.
14. Chen, C. L., Ip, S. M., Cheng, D., Wong, L. C. & Ngan, H. Y. (2000) Clin. Cancer Res. 6, 3910-3915.
15. Irwin, M., Mar, M. C., Phillips, A. C., Seelan, R. S., Smith, D. I., Liu, W., Flores, E. R., Tsai, K. Y., Jacks, T., Vousden, K. H. & Kaclin, W. G. Jr. (2000) Nature 407, 645-648.
16. Stiewe, T. & Putzer, B. M. (2000) Nat. Genet. 26,464-469.
17. Zaika, A., Irwin, M., Sansome, C. & Moll, U. M. (2001) J. Biol. Chem. 276, 11310-11316.
18. Pozniak, C. D., Radinovic, S., Yang, A., McKeon, F., Kaplan, D. R. & Miller, F. D. (2000) Science 289, 304-306.
19. Ozaki, T., Naka, M., Takada, N., Tada, M., Sakiyama, S. & Nakagawara, A. (1999) Cancer Res. 59, 5902-5 907.

### Industrial Applicability

As explained above, the present invention has elucidated the function of the ΔNp73 promoter, and allows regulation of p73 apoptosis-inducing activity by control of the activity of this promoter, p73 apoptosis-inducing activity may be utilized to accelerate apoptosis of tumor cells for an antitumor effect.

The invention further allows screening of p73 apoptosis regulators for tumor cells. Such apoptosis regulators are useful for chemotherapy, including gene therapy for tumors (particularly malignant tumors).

## Claims

1. A regulator for p73 apoptosis-inducing activity which utilizes heterooligomerization with ΔNp73.

2. A regulator for p53 apoptosis-inducing activity which utilizes heterooligomerization with ΔNp73.

3. A regulator for apoptosis-inducing activity which utilizes the base sequence set forth in SEQ ID NO: 1.

4. A regulator for apoptosis-inducing activity consisting of a nucleic acid comprising a base sequence which hybridizes to the base sequence set forth in SEQ ID NO: 1.

5. A p53 and p73 transactivation regulator comprising the p73 gene and the ΔNp73 gene.

6. A method for screening an apoptosis regulator in a tumor cell which utilizes a nucleic acid having the base sequence set forth in SEQ ID NO: 1.

7. A tumor cell apoptosis regulator obtainable by the method according to claim 6.
